(19) **Europäisches Patentamt / European Patent Office / Office européen des brevets**

(11) **EP 1 800 592 B1**

(12) **EUROPEAN PATENT SPECIFICATION**

(45) Date of publication and mention
of the grant of the patent:
**25.12.2013 Bulletin 2013/52**

(21) Application number: **04792497.2**

(22) Date of filing: **15.10.2004**

(51) Int Cl.:
***A61B 1/00*** *(2006.01)*

(86) International application number:
**PCT/JP2004/015280**

(87) International publication number:
**WO 2006/040831 (20.04.2006 Gazette 2006/16)**

(54) **CAPSULE-LIKE ENDOSCOPE SYSTEM AND CAPSULE-LIKE ENDOSCOPE**

KAPSELARTIGES ENDOSKOPSYSTEM UND KAPSELÄHNLICHES ENDOSKOP

SYSTÈME D'ENDOSCOPE À CAPSULE ET ENDOSCOPE À CAPSULE

(84) Designated Contracting States:
**DE FR GB**

(43) Date of publication of application:
**27.06.2007 Bulletin 2007/26**

(73) Proprietor: **Olympus Corporation**
**Tokyo 151-0072 (JP)**

(72) Inventors:
• **KIMOTO, Seiichiro**
**Tokyo 141-0072 (JP)**

• **SHIMIZU, Hatsuo**
**Tokyo 141-0072 (JP)**

(74) Representative: **von Hellfeld, Axel et al**
**Wuesthoff & Wuesthoff**
**Patent- und Rechtsanwälte**
**Schweigerstrasse 2**
**81541 München (DE)**

(56) References cited:
**WO-A-01/35813        WO-A-03/000122**
**JP-A- 5 137 693       JP-A- 2004 065 772**
**JP-A- 2004 167 008    US-A- 5 142 359**

## Description

TECHNICAL FIELD

The present invention relates to a swallowable medical endoscope system (hereinafter, "capsule endoscope system").

BACKGROUND ART

Recently, such a capsule endoscope system has been proposed that enables to observe a body cavity image by using a capsule endoscope that includes in a capsule-shaped case, an imaging unit including a solid-state image sensor such as a charge-coupled device (CCD), and an illuminating unit including a light emitter such as a light emitting diode (LED).

More specifically, when a subject swallows the capsule endoscope from a mouth, an image of the body cavity such as a stomach and an intestine is captured by the capsule endoscope while the capsule endoscope travels through inside the body cavity. An image data is transmitted to an external device outside the body cavity and visualized by using, for example, a monitor, to realize an observation of the body cavity image.

The image data acquired by the capsule endoscope is generated by performing a signal process such as a data correction (i.e., a color correction and a $\gamma$ correction) in the capsule endoscope or in a receiving device.

An image-data correction value used for the image-data correction is set in advance in accordance with a type of a light source that structures the illuminating unit or with a position of an imaging lens in the imaging unit.

WO 01/35813 A1 discloses a capsule-type medical device adapted to collect images. The capsule device is enclosed in a package inside a white balance cup such that, when an operator removes the capsule device from its packaging, the capsule remains inserted in the white balance cup. When the capsule is removed from the package containing a magnet preventing imaging and illumination devices inside the capsule from operating, the imaging and illumination devices are activated. Because the capsule remains inserted into the white balance cup, the first images are white, enabling automatic white balance. The images are processed to remove the influence of environmental light, wherein the white balance cup apparently is at least partially translucent. Finally the capsule can be snapped out of the white balance cup to be swallowed by the patient.

JP 2004167008 A discloses a capsule medical device which is activated by displacing a permanent magnet located inside the capsule medical device, thereby allowing a battery to power the capsule medical unit. The capsule medical device is placed in a white balance cup to perform a white balance procedure on the capsule medical device, and, thereafter, the capsule is swallowed by a patient. Images taken inside the patient's body are processed so as to achieve a white balance adjustment.

DISCLOSURE OF INVENTION

PROBLEM TO BE SOLVED BY THE INVENTION

However, because a color of the light source is slightly different and a position of the imaging lens varies, with respect to each product (capsule endoscope), there still is a possibility that an image visualized by performing a correction using the predetermined correction value does not have an optimal color reproduction and an optimal resolution in every capsule endoscope.

The image-data correction value can be acquired by capturing an image of a chart for acquiring the image-data correction value, before the capsule endoscope is introduced into the body cavity. However, when the above image is captured, an illumination in a room is likely to be input, and the image-data correction value obtained in the above situation is not suitable for capturing an image inside the body cavity, that is, not suitable for capturing an image with a light source of a light emitter in the capsule endoscope. Further, it is difficult to capture an image of the chart with the light source of the light emitter in the capsule endoscope.

An object of the present invention is to solve the above problems and to provide a capsule endoscope system that enables to easily acquire the most optimal image-data correction value with respect to each product (capsule endoscope) so that the visualized image has the optimal color reproduction and resolution, and further, to make it possible to verify whether the image-data correction value is most optimal.

MEANS FOR SOLVING PROBLEM

This problem is solved by a capsule endoscope system according to claim 1

An advantage of the capsule endoscope system according to the present invention is that the image-data correction value becomes optimal. Further, the optimal image-data correction value can easily be acquired and power supply can be initiated to the capsule endoscope at a substantially same timing. Moreover, it becomes possible to verify whether the correction value is optimal, by visualizing a secondly captured image by the display unit.

EFFECT OF THE INVENTION

With a capsule endoscope system according to the present invention, an image-data correction value is acquired in the capsule endoscope with respect to each product before the capsule endoscope is introduced into the body cavity, in such a condition that an imaging window and an illumination window are covered by a package. Accordingly, the most optimal image-data correction value can be acquired, and further, it becomes possible to easily acquire the most optimal image-data correction value. Moreover, it becomes possible to verify whether the image-data correction value is most optimal, by visualizing a secondly captured image by a display unit.

BRIEF DESCRIPTION OF DRAWINGS

FIG. 1 is a schematic view of a configuration of a capsule endoscope system according to an embodiment of the present invention;
FIG. 2 is a sectional side view of a capsule endoscope according to the embodiment of the present invention;
FIG. 3 is a block diagram of the capsule endoscope system according to the embodiment of the present invention;
FIG. 4 is a sectional side view of an external package, an inner package, and a chart portion according to the embodiment of the present invention;
FIG. 5 is a front view of the chart portion according to the embodiment of the present invention;
FIG. 6 is a flowchart of a process procedure of the capsule endoscope system according to the embodiment of the present invention; and
FIG. 7 is a schematic view for describing a chart gradation of a gray chart according to the embodiment of the present invention.

EXPLANATIONS OF LETTERS OR NUMERALS

1    Capsule endoscope system
2    Capsule endoscope
4    External device
4a   Receiving device
50   Package
51   External package
70   Internal package
71   Chart portion

BEST MODE(S) FOR CARRYING OUT THE INVENTION

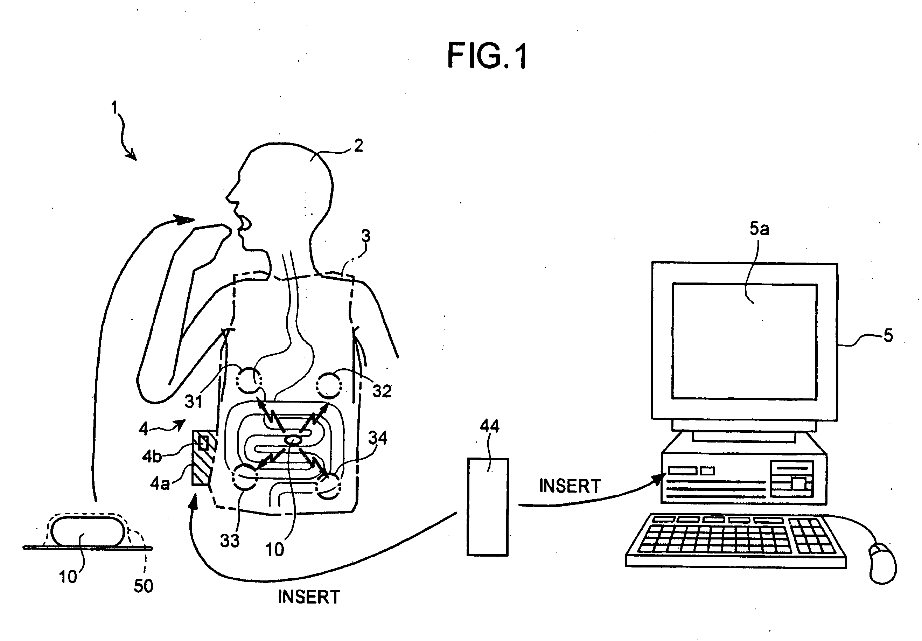

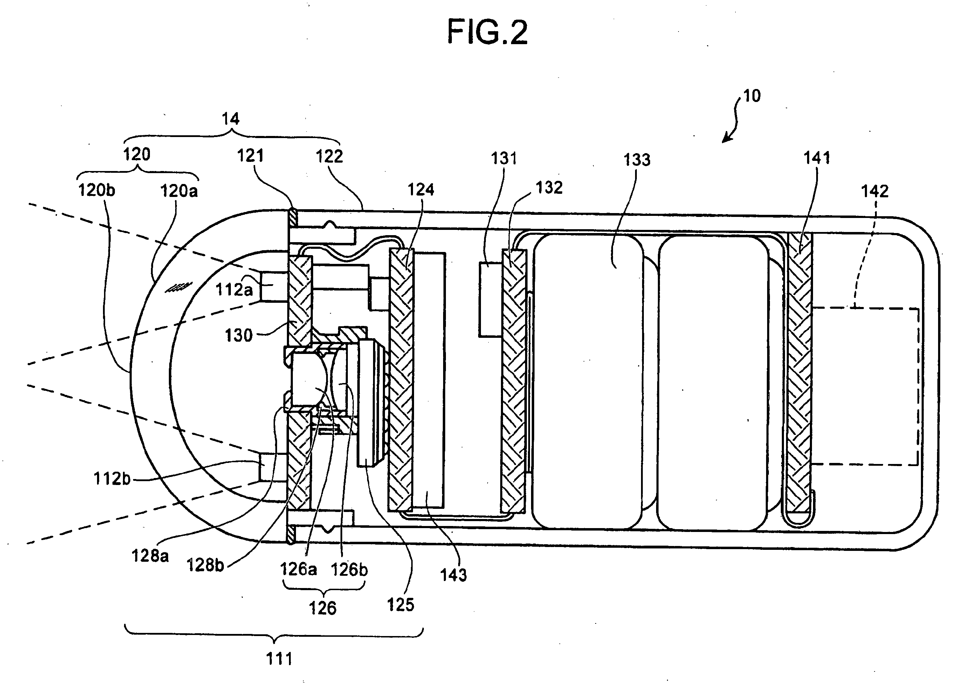

Exemplary embodiments of the present invention will be described below. FIG. 1 is a schematic view of a configuration of a capsule endoscope system according to an embodiment of the present invention. FIG. 2 is a sectional side view of a capsule endoscope for use in a system according to the embodiment of the present invention.

The capsule endoscope system according to the embodiment of the present invention includes, as shown in FIG. 1, a capsule endoscope 10 that is swallowable from a mouth and captures an image of a body cavity in a process that the capsule endoscope 10 passes through the body cavity such as an esophagus, a stomach, a small intestine, and a colon, and includes an external device 4 that is physically and entirely separated from the capsule endoscope 10 to receive, by a radio communication, image data acquired by the capsule endoscope 10.

The capsule endoscope 10 includes, as shown in FIG. 2, a capsule main body 14 that is a capsule-shaped exterior package, an imaging unit 111 that is arranged inside the capsule main body 14 and captures an image, illuminating units 112a and 112b that illuminate an imaging target region, and a control unit 143 that performs a control of the imaging unit 111 and the illuminating units 112a and 112b or performs a signal process.

For describing each of the units in detail, the capsule main body 14 is in a size swallowable by a human and formed by watertightly bonding a substantially hemisphere-shaped tip portion 120 to a cylindrical-shaped body portion 122. The tip portion 120 and the body portion 122 make the tip portion 120 and the body portion 122 be watertightly bonded via an O-ring 121. It is needles to say that the shape and the configuration of the capsule main body 14 are not limited to those in the present embodiment.

The imaging unit 111 includes a solid-state image sensor 125 arranged on an imaging board 124 and an imaging lens 126 that forms an image of a subject image on the solid-state image sensor 125. A CCD is used as the solid-state image sensor 125. It is obvious that the solid-state image sensor 125 is not limited to the CCD and can be a complementary metal-oxide semiconductor (CMOS). The imaging lens 126 includes a first lens 126a arranged on a fixed frame 128a and positioned on the side of the subject, and a second lens 126b arranged on a movable frame 128b and positioned on the side of the CCD. The imaging lens 126 performs a focus adjustment by shifting the second lens 126b backward and forward. A transparent imaging window 120b is formed on the tip portion 120 of the capsule main body 14.

The illuminating units 112a and 112b are formed by arranging a light emitting diode (LED) as a light emitter on an illuminating board 130. Particularly, according to the present embodiment, four light emitters are arranged in a surrounding area of the imaging lens 126. A transparent illumination window 120a is formed on the tip portion 120 of the capsule endoscope 10 so that a light emitted from the light emitter is output to the outside of the capsule main body 14. Further, according to the present embodiment, the entire tip portion 120 of the capsule endoscope 10 is made of a transparent resign, and the illumination window 120a and the imaging window 120b are partly overlapped. It is obvious that the illumination window 120a and the imaging window 120b can be clearly separated from each other in the configuration.

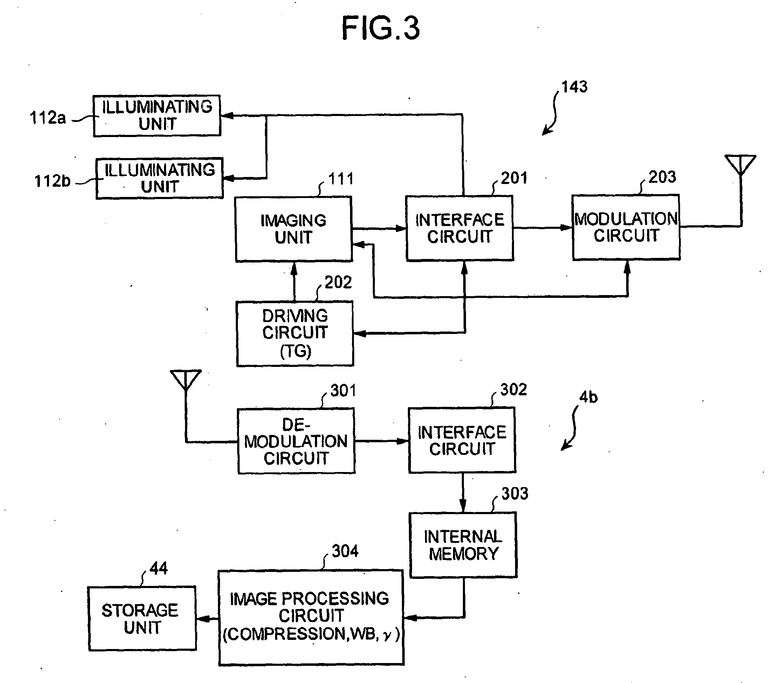

A configuration of the control unit 143 will be explained with reference to FIG. 3. FIG. 3 is a block diagram of the capsule endoscope system according to the present embodiment. Specifically, FIG. 3 describes a flow of image data from the control unit 143 in the capsule endoscope 10 to a receiving-device main body 4b in the external device 4. The control unit 143 includes an interface circuit 201 and a driving circuit 202, performs various signal processes of, for example, generating image data in cooperation with the imaging unit 111, performs a timing-generator function of enabling the imaging unit 111 to capture an image at a predetermined time interval, and stores various parameters related to, for example, a line or a frame. Therefore, according to the present embodiment, two images are.captured per second by the imaging unit 111, image data is generated by performing the signal process such as a color correction, and the image data is sequentially transmitted by radio to the external device 4.

A radio transmission is performed, as shown in FIG. 2 and FIG. 3, by a radio transmitting unit configured by providing an antenna on a radio board 141. When the radio transmission is performed, the image data is transmitted and received between the capsule endoscope 10 and the external device 4 by a modulation circuit 203 arranged in the capsule endoscope 10 and a demodulation circuit 301 arranged in the external device 4, using a predetermined communication method (i.e., phase shift keying (PSK), minimum shift keying (MSK), gaussian minimum shift keying (GMSK), quadrature minimum shift keying (QMSK), and amplitude shift keying (ASK)).

On the other hand, as shown in FIG. 1, the external device 4 includes a receiving device 4a that includes antennas 31, 32, 33, and 34 arranged on proper positions of a chest or a abdomen of a subject 2 and the receiving-device main body 4b.

The antennas 31, 32, 33, and 34 are connected to the receiving-device main body 4b with wires. The antennas 31, 32, 33, and 34 are arranged inside a jacket 3 that is wearable on an upper body of the subject 2. Particularly, according to the present embodiment, the antennas 31, 32, 33, and 34 are arranged inside the jacket 3 and an antenna with the highest receiver sensitivity corresponding to a movement of the capsule endoscope inside the body cavity can be selected by a selector arranged in the receiving-device main body 4b to receive the image data. The jacket 3 is an example of the present embodiment, and the present invention is not thus limited.

As shown in FIG. 3, the receiving-device main body 4b includes the interface circuit 302, an internal memory 303, an image processing circuit 304 (the image processing circuit 304 according to the present embodiment includes a function as a control unit explained later, however, it is acceptable to configure the image processing circuit 304 and the control unit independently). The receiving-device main body 4b performs the signal process of, for example, data reproduction or data compression for image data received by a predetermined communication method, and stores the image data in a storage unit 44. As the signal process, a color correction, a γ correction, and a color process are performed. The storage unit 44 is configured by connecting a removable storage medium such as a compact flash (CF) memory card or a memory stick to the receiving-device main body 4b. As shown in FIG. 1, by causing an information processing device 5 such as a personal computer to read the storage medium, a body cavity image visualized from the image data stored in the storage medium can be displayed on a display unit 5a such as a monitor.

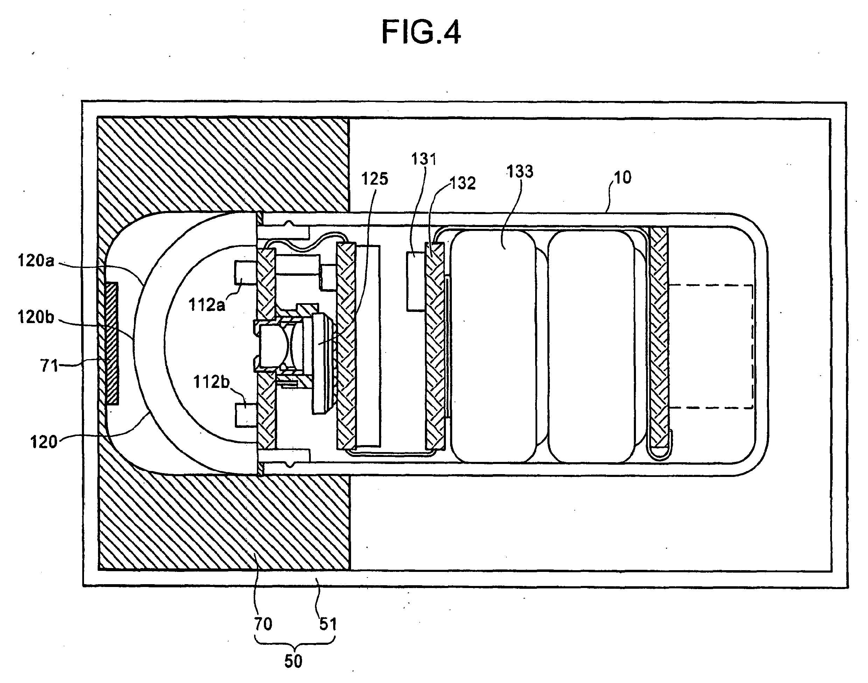

Therefore, the capsule endoscope system 1 according to the present embodiment includes, as shown in FIG. 4, a package 50 that covers the imaging window 120b and the illumination window 120a of the capsule endoscope 10, a power-supply initiating unit that causes the capsule endoscope 10 to initiate power supply in such a state that the imaging window 120b and the illumination window 120a are covered by the package 50, and a chart portion 71 arranged inside the package 50 so that the chart portion 71 faces the imaging window 120b.

Each of the units will be described below in detail. The package 50 includes an external package 51 in which the capsule endoscope 10 is installed and an internal package 70 that is arranged inside the external package 51 and covers the capsule endoscope 10.

The external package 51 enables to store the capsule endoscope 10 in such a state that the capsule endoscope 10 is shielded from the outside before the capsule endoscope 10 is introduced inside the body of the subject 2. Although the external package 51 having a rectangular-shaped cross section is used as shown in the drawings, the present invention is not thus limited.

On the other hand, the internal package 70 is made of a soft material having a light blocking effect. Further, the internal package 70 completely covers the illumination window 120a and the imaging window 120b of the capsule endoscope 10 to prevent a light from the outside from irradiating the illumination window 120a and the imaging window 120b, when the capsule endoscope 10 is removed from the external package 51. The internal package 70 is removably set on the tip portion 120 of the capsule main body 14 so that a relative position between the chart portion 71 and the capsule main body 14 is kept constant.

The power-supply initiating unit is for initiating to start supplying power to, for example, the imaging unit 111 and the illuminating unit 112, specifically in such a state that the external package 51 is removed and the internal package 70 covers the illumination window 120a and the imaging window 120b of the capsule endoscope 10. The power-supply initiating unit includes an ON/OFF switch 131, an external magnet, and an internal magnet.

The ON/OFF switch 131 is arranged on a power board unit 132 of the capsule endoscope 10 for causing the battery 133 (i.e., silver oxide battery), which is also arranged on the power board unit 132, to start supplying power to the capsule endoscope 10.

The external magnet is arranged in the package 50 in which the capsule endoscope 10 is installed to energize the ON/OFF switch 131 to an OFF state.

On the other hand, the internal magnet is arranged in a neighboring area of the ON/OFF switch 131 inside the capsule main body 14 to energize the ON/OFF switch 131 to an ON state. Therefore, it is configured so that when the capsule endoscope 10 is separated from the external magnet by, for example, removing the capsule endoscope 10 from the package 50, the ON/OFF switch 131 in the OFF state is switched to the ON state. The switch mechanism is not limited to the above described ones. As for the battery 133, a rechargeable battery or an external-power supply type accumulator can be acceptable.

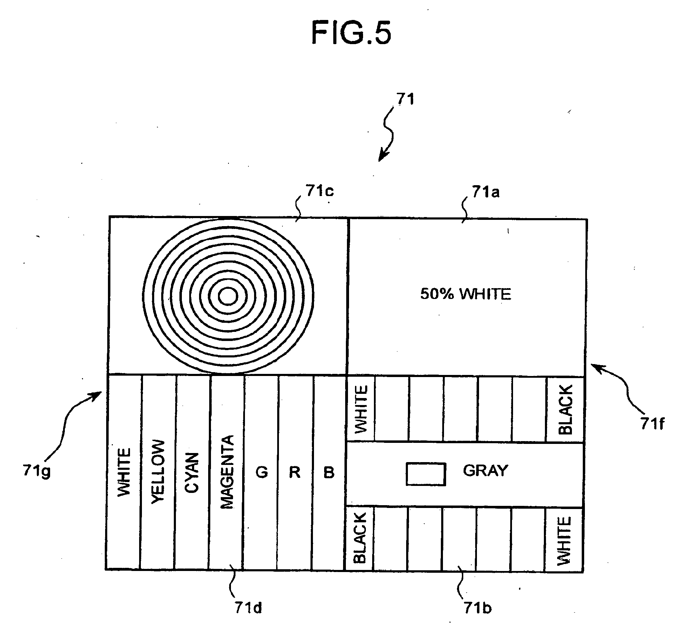

The chart portion 71 includes a chart portion 71f for acquiring an image-data correction value and a chart portion 71g for a verification, and are arranged inside the internal package 70. The chart portion 71 is in a substantially rectangular-plate shape and includes the chart portion 71f for acquiring the image-data correction value and the chart portion 71g for the verification, on a surface at a side on which the imaging window 120b is arranged. Specifically, according to the present embodiment, the chart portion 71f for acquiring the image-data correction value includes a chart for acquiring a color-correction value and a chart for acquiring a gradation-correction value, while the chart portion 71g for the verification includes a resolution chart and a color bar. As shown in FIG. 5, the chart portion 71 is divided at each of middle positions on a vertical and a horizontal side, resulting in being divided into four areas 71a to 71d. The first area 71a is the chart for acquiring the color-correction value, specifically, according to the present embodiment, a white chart (50% white) for acquiring a white-balance correction value. The second area 71b is the chart for acquiring the gradation-correction value, specifically, according to the present embodiment, a gray scale chart. The gray scale chart has an upper column, a middle column, and a lower column. In the upper column, colors corresponding to each of brightness (gradation) from white to black are displayed at, for example, 10 levels from the left side to the right side of the chart. In the middle column, solid gray is displayed. In the lower column, colors corresponding to each of brightness (gradation) from black to white are displayed at, for example, 10 levels from the left side to the right side of the chart. The third area 71c is the resolution chart as the chart for the verification. More specifically, the third area 71c is a so-called circular zone chart in which a plurality of concentric circles is formed with an arrangement in which the concentric circles become denser toward an outer circumference. The fourth area 71d is the color bar as the chart for the verification, in which white, yellow, cyan, magenta, green (G), red (R), and blue (B) are displayed in that order from the left side of the color bar. A layout of arranging each of the white chart, the gray scale chart, the resolution chart, and the color bar is not limited to one described in the present embodiment and various modification can be acceptable, for example, the color bar can be arranged in the center.

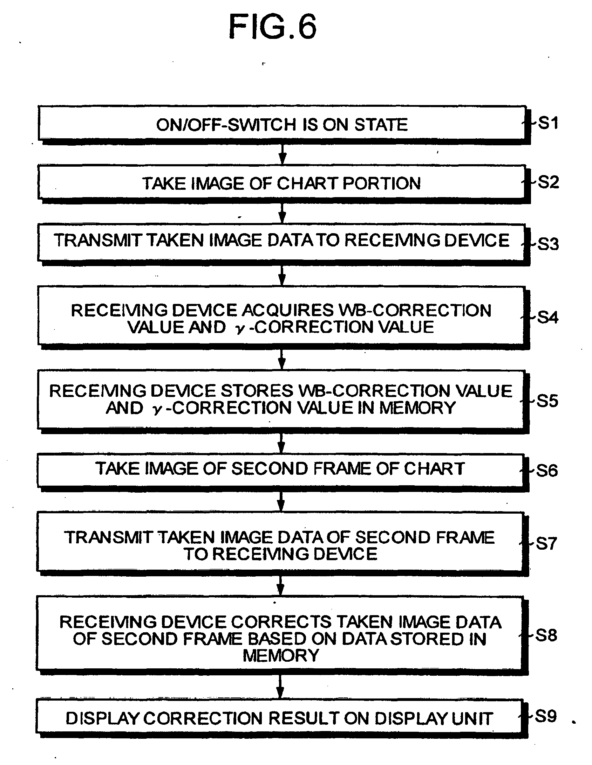

An operation of the capsule endoscope system according to the present embodiment is explained with reference to FIG. 6. The capsule endoscope 10 is removed from the external package 51. At the same time, the ON/OFF switch is switched to the ON state (step S1).

Accordingly, a power is supplied from the battery 133 to the radio unit including the imaging unit 111, the illuminating units 112a and 112b, an antenna 142, and the radio board 141, and a light emitter emits a light. On the other hand, because the imaging unit 111 is configured, as described above, to capture an image of the chart portion 71 illuminated by the illuminating units 112a and 112b at a predetermined time interval, the imaging unit 111 performs a first imaging of the chart portion 71 fixed to the internal package 70 when the predetermined time passed (step S2).

The image data is transmitted to the receiving device 4a via the radio unit including the radio board 141 and the antenna 142 (step S3).

When the receiving device 4a receives the image data of the chart portion 71, the image processing circuit 304 that performs a function as the control unit acquires a white balance (WB)-correction value used for a white-balance correction from the white chart, based on the image data, and acquires a $\gamma$-correction value used for a gradation ($\gamma$) correction from the gray scale chart (step S4). Thereafter, the image processing circuit 304 stores the WB-correction value and the $\gamma$-correction value in the internal memory 303 of the receiving-device main body 4b (step S5).

At this state, values $\alpha$ and $\beta$ as the WB-correction values are obtained as the following. Namely, each of signal levels (brightness) corresponding to R, G, and B in a predetermined area (i.e., an area of 16x16) of the chart portion used for acquiring the WB-correction value is extracted, and $\alpha$ and $\beta$ are obtained so that $\alpha$ and $\beta$ satisfy each of equations,

(average of signal level of G)=$\alpha$(average of signal level of R), and

(average of signal level of G)=$\beta$(average of signal level of B).

Further, the $\gamma$-correction value is determined by the following Equation.

## [Equation. 1]

$$Y=X^{\gamma}$$

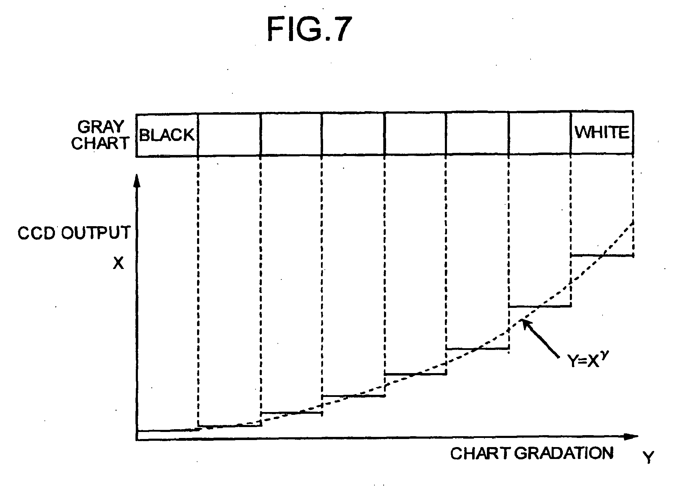

Namely, $\gamma$ is determined by performing, for example, a fitting from values of X and Y so that the above equation becomes true. At this state, as shown in FIG. 7, Y is the chart gradation determined by the gray chart, while X is a CCD output.

The values $\alpha$, $\beta$, and $\gamma$ can be obtained by other ways.

Thereafter, the capsule endoscope 10 performs a second imaging of the chart portion 71 when the predetermined time passed (step S6), and transmits the image data to the receiving device 4a (step S7).

When the receiving device 4a receives the image data of the second imaging, the image processing circuit 304 that performs a function as the control unit performs the WB-correction and the γ-correction to the image data based on the correction value α, β, and γ (step S8), and stores the processed image data in the storage unit 44.

The image data stored in the storage unit 44 at step S8 is visualized and displayed by the display unit 5a, by causing the information processing device 5 to read the image data (step S9). Accordingly, the image displayed on the display unit 5a can be viewed and it becomes possible to confirm that the correction has been properly performed, by checking an image corresponding to the fourth area (color bar) 71d or an image corresponding to the third area (resolution chart) 71c included in the displayed image.

INDUSTRIAL APPLICABILITY

As described, the capsule endoscope system according to the present invention is suitable for, i.e., a swallowable capsule endoscope used in a medical field.

**Claims**

**1.** A capsule endoscope system (1) comprising:

a capsule endoscope (10) containing an imaging unit (111), an illuminating unit (112a, 112b) capable of illuminating an imaging target region, and a transmitting unit (141, 142) capable of transmitting image data obtained by the imaging unit (iii) to an outside;
an external device (4) separated from the capsule endoscope (10), the external device (4) including a receiving unit (4a) configured to receive the image data and a display unit (5a) capable of visualizing and displaying the image captured by the imaging unit (111);
a package (50) configured to cover an imaging window (120b) and an illumination window (120a) of the capsule endoscope (10) such that light from the outside is prevented from irradiating the imaging window (120b) and the illumination window (120a);
a power-supply initiating unit (131) capable of initiating a power supply to the imaging unit (iii) and the illuminating unit (112a, 112b) in a state wherein the imaging window (120b) and the illumination window (120a) are covered by the package (50); and
a chart portion (71) which is arranged inside the package (50) so that the chart portion (71) faces the imaging window (120b), and which includes a first subportion (71f) adapted for acquiring an image-data correction value;
wherein the system (i) is adapted to acquire the image-data correction value from image data of the first subportion (71f) of the chart portion (71), which is obtained by performing a first imaging of the chart portion (71), in such a state wherein the imaging window (120b) and the illumination window (120a) are covered by the package (50), wherein the chart portion (71) further includes a second subportion (71g) adapted for verifying the image-data correction value acquired using the first subportion (71f) of the chart portion (71), and wherein the system (1) further is adapted to correct image data of the second subportion (71g) of the chart portion (71), which is obtained by performing a second imaging of the chart portion (71) after the first imaging, by the image-data correction value, in a state wherein the imaging window (120b) and the illumination window (120a) are covered by the package (50), and to visualize and display the corrected image data of the second subportion (71g) of the chart portion (71) on the display unit (5a),
the package (50) includes an external package (51) adapted to accommodate therein a capsule main body (14), and an internal package (70) that is arranged inside the external package (51) and is adapted to cover the imaging window (120b) and the illumination window (120a) of the capsule main body (14),
the chart portion (71) for acquiring the image-data correction value is arranged inside the internal package (70), and
the internal package (70) is removably set on the tip portion of the capsule endoscope (10) to keep a relative position between the chart portion (71) and the capsule main body (14) constant.

**2.** The capsule endoscope system (1) according to claim 1, wherein the power-supply initiating unit (131) is adapted to initiate power supply when the external package (51) is removed.

**3.** The capsule endoscope system (1) according to claim 1, wherein the first subportion (71f) of the chart portion (71) includes a chart (71a) for acquiring a color-correction value and the chart (71a) is a white chart.

**4.** The capsule endoscope system (1) according to claim 1, wherein the first subportion (71f) of the chart portion (71) includes a chart (71b) for acquiring a gradation-correction value and the chart (71b) is a gray scale chart.

**5.** The capsule endoscope system (1) according to claim 1, wherein the second subportion (71g) of the chart portion (71) includes a color bar portion (71d) and a resolution chart portion (71c).

**6.** The capsule endoscope system (1) according to claim 6, wherein the first and the second subportion (71f, 71g) of the chart portion (71) are arranged on the same member.

## Patentansprüche

**1.** Kapselendoskopsystem (1) mit:

einem Kapselendoskop 10), das eine Bildgebungseinheit (111), eine Beleuchtungseinheit (112a, 112b), die eine Bildgebungs-Zielregion zu beleuchten vermag, und eine Sendeeinheit (141, 142) aufweist, die durch die Bildgebungseinheit (111) gewonnen Bilddaten nach außen zu senden vermag;

einer Außenvorrichtung (4), die vom Kapselendoskop (10) getrennt ist, wobei die Außenvorrichtung (4) eine Empfangseinheit (4a), die zum Empfangen der Bilddaten ausgebildet ist, und eine Anzeigeeinheit (5a) enthält, die das von der Bildgebungseinheit (111) erfasste Bild zu visualisieren und anzuzeigen vermag;

einer Verpackung (50), die so zum Bedecken eines Bildgebungsfensters (120b) und eines Beleuchtungsfensters (120a) des Kapselendoskops (10) ausgebildet ist, dass Licht von außen daran gehindert wird, das Bildgebungsfenster (120b) und das Beleuchtungsfenster (120a) zu bestrahlen;

einer Stromversorgungs-Initialeinheit (131), die eine Stromversorgung für die Bildgebungseinheit (111) und die Beleuchtungseinheit (112a, 112b) in einem Zustand in Gang zu setzen vermag, in dem das Bildgebungsfenster (120b) und das Beleuchtungsfenster (120a) durch die Verpackung (50) bedeckt sind; und

einem Grafikbereich (71), der im Innern der Verpackung (50) so angeordnet ist, dass der Grafikbereich (71) dem Bildgebungsfenster (120b) zugewandt ist, und der einen ersten Unterbereich (71f) aufweist, der zum Erfassen eines Bilddaten-Korrekturwerts ausgebildet ist;

wobei das System (1) dazu ausgebildet ist, den Bilddaten-Korrekturwert aus Bilddaten des ersten Unterbereichs (71f) des Grafikbereichs (71) zu erfassen, die durch Ausführen einer ersten Bildgebung des Grafikbereichs (71) in einem Zustand gewonnen werden, in dem das Bildgebungsfenster (120b) und das Beleuchtungsfenster (120a) durch die Verpakkung (50) bedeckt sind,

wobei der Grafikbereich (71) des Weiteren einen zweiten Unterbereich (71g) aufweist, der zum Überprüfen des Bilddaten-Korrekturwerts ausgebildet ist, welcher unter Verwendung des ersten Unterbereichs (71f) des Grafikbereichs (71) erfasst wird, und wobei das System (1) des Weiteren dazu eingerichtet ist, Bilddaten des zweiten Unterbereichs (71g) des Grafikbereichs (71), welche nach der ersten Bildgebung durch Ausführen einer zweiten Bildgebung des Grafikbereichs (71) gewonnen werden, durch den Bilddaten-Korrekturwert in einem Zustand zu korrigieren, in dem das Bildgebungsfenster (120b) und das Beleuchtungsfenster (120a) durch die Verpackung (50) bedeckt sind, und die korrigierten Bilddaten des zweiten Unterbereichs (71g) des Grafikbereichs (71) auf der Anzeigeeinheit (5a) zu visualisieren und anzuzeigen,

die Verpackung (50) eine äußere Verpackung (51), die dazu ausgebildet ist, einen Kapselhauptkörper (14) aufzunehmen, und eine innere Verpackung (70) aufweist, die im Innern der äußeren Verpackung (51) angeordnet und dazu ausgebildet ist, das Bildgebungsfenster (120b) und das Beleuchtungsfenster (120a) des Kapselhauptkörpers (14) zu bedecken,

der Grafikbereich (71) zum Erfassen des Bilddaten-Korrekturwerts im Innern der inneren Verpackung (70) angeordnet ist, und

die innere Verpackung (70) abnehmbar am Spitzenbereich des Kapselendoskops (10) fixiert ist, um eine relative Position zwischen dem Grafikbereich (71) und dem Kapselhauptkörper (14) konstant zu halten.

**2.** Kapselendoskopsystem (1) nach Anspruch 1, wobei die Stromversorgungs-Initialeinheit (131) dazu ausgebildet ist, die Stromversorgung in Gang zu setzen, wenn die äußere Verpackung (51) entfernt wird.

**3.** Kapselendoskopsystem (1) nach Anspruch 1, wobei der erste Unterbereich (71f) des Grafikbereichs (71) eine Grafik (71a) zum Erfassen eines Farb-Korrekturwerts aufweist und die Grafik (71a) eine Weiß-Grafik ist.

**4.** Kapselendoskopsystem (1) nach Anspruch 1, wobei der erste Unterbereich (71f) des Grafikbereichs (71) eine Grafik (71b) zum Erfassen eines Gradations-Korrekturwerts aufweist und die Grafik (71b) eine Graustufen-Grafik ist.

**5.** Kapselendoskopsystem (1) nach Anspruch 1, wobei der zweite Unterbereich (71g) des Grafikbereichs (71) einen Farbbalken-Bereich (71d) und einen Auflösungsgrafik-Bereich (71c) aufweist.

**6.** Kapselendoskopsystem (1) nach Anspruch 6, wobei der erste und der zweite Unterbereich (71f, 71g) des Grafikbereichs (71) auf demselben Element angeordnet sind.

## Revendications

**1.** Système d'endoscope de type capsule (1) comprenant :

un endoscope de type capsule (10) contenant une unité d'imagerie (111), une unité d'éclairage (112a, 112b) capable d'éclairer une région cible d'imagerie, et une unité de transmission (141, 142) capable de transmettre des données d'image obtenues par l'unité d'imagerie (lil) vers l'extérieur ;
un dispositif externe (4) séparé de l'endoscope de type capsule (10), le dispositif externe (4) incluant une unité de réception (4a) configurée pour recevoir les données d'image et une unité d'affichage (5a) capable de visualiser et d'afficher l'image capturée par l'unité d'imagerie (111) ;
un boîtier (50) configuré pour couvrir une fenêtre d'imagerie (120b) et une fenêtre d'éclairage (120a) de l'endoscope de type capsule (10) d'une manière telle qu'une lumière provenant de l'extérieur est empêchée d'irradier la fenêtre d'imagerie (120b) et la fenêtre d'éclairage (120a) ;
une unité d'initiation d'alimentation (131) capable d'initier une alimentation vers l'unité d'imagerie (111) et l'unité d'éclairage (112a, 112b) dans un état dans lequel la fenêtre d'imagerie (120b) et la fenêtre d'éclairage (120a) sont couvertes par le boîtier (50) ; et
une partie de diagramme (71) qui est disposée à l'intérieur du boîtier (50) de sorte que la partie de diagramme (71) est en regard de la fenêtre d'imagerie (120b), et qui inclut une première sous-partie (71f) adaptée pour acquérir une valeur de correction de données d'image ;
dans lequel le système (1) est adapté pour acquérir la valeur de correction de données d'image à partir des données d'image de la première sous-partie (71f) de la partie de diagramme (71), qui sont obtenues en exécutant une première imagerie de la partie de diagramme (71), dans un état dans lequel la fenêtre d'imagerie (120b) et la fenêtre d'éclairage (101a) sont couvertes par le boîtier (50),
dans lequel la partie de diagramme (71) inclut en outre une deuxième sous-partie (71g) adaptée pour vérifier la valeur de correction de données d'image acquises en utilisant la première sous-partie (71f) de la partie de diagramme (71), et dans lequel le système (1) est en outre adapté pour corriger des données d'image de la deuxième sous-partie (71g) de la partie de diagramme (71), qui sont obtenues en exécutant une deuxième imagerie de la partie de diagramme (71) après la première imagerie, par la valeur de correction de données d'image, dans un état dans lequel la fenêtre d'imagerie (120b) et la fenêtre d'éclairage (120a) sont couvertes par le boîtier (50), et pour visualiser et afficher les données d'image corrigées de la deuxième sous-partie (71g) de la partie de diagramme (71) de l'unité d'affichage (5a),
le boîtier (50) inclut un boîtier externe (51) adapté pour recevoir dans celui-ci un corps principal de capsule (14), et un boîtier interne (70) qui est disposé à l'intérieur du boîtier externe (51) et est adapté pour couvrir la fenêtre d'imagerie (120b) et la fenêtre d'éclairage (120a) du corps principal de capsule (14),
la partie de diagramme (71) destinée à acquérir la valeur de correction de données d'image est disposée à l'intérieur du boîtier interne (70), et le boîtier interne (70) est fixé de manière amovible sur la partie de pointe de l'endoscope de type capsule (10) pour maintenir une position relative entre la partie de diagramme (71) et le corps principal de capsule (14) constante.

**2.** Système d'endoscope de type capsule (1) selon la revendication 1, dans lequel l'unité d'initiation d'alimentation (131) est adaptée pour initier l'alimentation lorsque le boîtier externe (51) est enlevé.

**3.** Système d'endoscope de type capsule (1) selon la revendication 1, dans lequel la première sous-partie (71f) de la partie de diagramme (71) comprend un diagramme (71a) destiné à acquérir une valeur de correction de couleur et le diagramme (71a) est un diagramme des blancs.

**4.** Système d'endoscope de type capsule (1) selon la revendication 1, dans lequel la première sous-partie (71f) de la partie de diagramme (71) comprend un diagramme (71b) destiné à acquérir une valeur de correction de gradation et le diagramme (71b) est un diagramme d'échelle de gris.

**5.** Système d'endoscope de type capsule (1) selon la revendication 1, dans lequel la deuxième sous-partie (71g) de la partie de diagramme (71) comprendre une partie de barre de couleurs (71d) et une partie de diagramme de résolution (71c).

**6.** Système d'endoscope de type capsule (1) selon la revendication 5, dans lequel la première et la deuxième sous-parties (71f, 71g) de la partie de diagramme (71) sont disposées sur le même élément.

# FIG.1

EP 1 800 592 B1

# FIG.2

EP 1 800 592 B1

# FIG.3

ILLUMINATING UNIT — 112a

ILLUMINATING UNIT — 112b

IMAGING UNIT — 111

INTERFACE CIRCUIT — 201

MODULATION CIRCUIT — 203

DRIVING CIRCUIT (TG) — 202

143

DE-MODULATION CIRCUIT — 301

INTERFACE CIRCUIT — 302

4b

INTERNAL MEMORY — 303

IMAGE PROCESSING CIRCUIT (COMPRESSION, WB, γ) — 304

STORAGE UNIT — 44

EP 1 800 592 B1

FIG.4

# FIG.5

# FIG.6

| | |
|---|---|
| ON/OFF-SWITCH IS ON STATE | S1 |
| TAKE IMAGE OF CHART PORTION | S2 |
| TRANSMIT TAKEN IMAGE DATA TO RECEIVING DEVICE | S3 |
| RECEIVING DEVICE ACQUIRES WB-CORRECTION VALUE AND $\gamma$-CORRECTION VALUE | S4 |
| RECEIVING DEVICE STORES WB-CORRECTION VALUE AND $\gamma$-CORRECTION VALUE IN MEMORY | S5 |
| TAKE IMAGE OF SECOND FRAME OF CHART | S6 |
| TRANSMIT TAKEN IMAGE DATA OF SECOND FRAME TO RECEIVING DEVICE | S7 |
| RECEIVING DEVICE CORRECTS TAKEN IMAGE DATA OF SECOND FRAME BASED ON DATA STORED IN MEMORY | S8 |
| DISPLAY CORRECTION RESULT ON DISPLAY UNIT | S9 |

# FIG.7

GRAY CHART | BLACK | | | | | | | WHITE

CCD OUTPUT
X

CHART GRADATION    Y

$Y=X^{\gamma}$

**EP 1 800 592 B1**

**Patent documents cited in the description**

- WO 0135813 A1
- JP 2004167008 A